(19) Europäisches Patentamt
European Patent Office
Office européen des brevets

(11) Numéro de publication: **0 138 635**
**A2**

(12) **DEMANDE DE BREVET EUROPEEN**

(21) Numéro de dépôt: **84401601.4**

(22) Date de dépôt: **31.07.84**

(51) Int. Cl.[4]: **A 61 L 9/01**, A 61 L 9/04

(30) Priorité: **23.09.83 FR 8315208**

(43) Date de publication de la demande: **24.04.85**
**Bulletin 85/17**

(84) Etats contractants désignés: **BE CH DE GB IT LI LU NL SE**

(71) Demandeur: **SOCIETE EPARCO S.A., 78, rue de Provence, F-75009 Paris (FR)**

(72) Inventeur: **Bavaveas, Tristan, 56, Avenue de Suffren, F-75015 Paris (FR)**

(74) Mandataire: **Derambure, Christian, Cabinet BUGNION ASSOCIES SARL 116, boulevard Haussmann, F-75008 Paris (FR)**

(54) **Procédé de fabrication d'une composition parfumée, et composition obtenue par ce procédé.**

(57) L'invention concerne l'industrie de la parfumerie.

Le procédé de fabrication d'une composition parfumée est telle que l'on solubilise une essence de parfum, de préférence une essence naturelle, dans un mélange d'alcool et d'un émulgateur, puis, on ajoute de l'eau en vue de réaliser une solution aqueuse parfumée homogène et stable. La solution ainsi obtenue comprend outre l'essence naturelle de parfum, l'émulgateur et l'alcool, un colorant acide n'ayant pas d'affinité pour l'émail. L'émulgateur est de préférence un octyl phénol à dix moles d'oxyde d'éthylène et l'alcool est de préférence l'alcool éthylique.

Application à une composition parfumée utilisable dans des chasses d'eau de toilettes.

EP 0 138 635 A2

PROCEDE DE FABRICATION D'UNE COMPOSITION PARFUMEE ET COMPOSITION OBTENUE PAR CE PROCEDE.

La présente invention concerne une composition parfumée nouvelle, pouvant être utilisée notamment pour des chasses d'eau de toilettes.

On connaît divers produits qui sont actuellement dans le commerce, destinés à être utilisés dans les toilettes. Il s'agit en premier lieu de blocs solides disposés dans la pièce même des toilettes, constitués le plus souvent de naphtaline et d'un parfum, ce dernier se diffusant au cours du temps dans l'atmosphère de la pièce. En second lieu, on connaît des produits liquides disposés dans un réservoir placé lui-même dans le réservoir d'eau de la chasse d'eau et dont une faible quantité s'écoule en même temps que l'eau contenue dans le réservoir. Ces produits liquides pour chasse d'eau sont constitués d'une solution dans l'eau, d'un parfum ou mélange de parfums, d'un colorant et d'un nettoyant tel qu'un produit moussant et éventuellement d'un agent séquestrant, à savoir un agent formant un complexe avec les ions $Ca^{++}$. On connaît enfin les blocs javellisants placés directement dans la cuvette.

En général, les colorants préférés pour ces produits sont des colorants anioniques ou acides, car, contrairement

aux colorants basiques, ils ne tachent pas l'émail de la cuvette des toilettes, n'ayant pas d'affinité avec l'émail.

L'agent moussant est par exemple dans des proportions allant de 1 à 2 % en poids de la solution aqueuse. De tels produits connus présentent cependant des inconvénients. Au cours du temps, il se produit un relargage du ou des parfums en surface et la solution aqueuse n'est pas homogène. L'agent moussant est un tensio-actif anionique et il a aussi des propriétés nettoyantes. Or,les cuvettes de toilettes sont fréquemment le siège d'une prolifération de micro-organismes indésirables. Par ailleurs, ces produits ne parfument que l'eau des toilettes et pour parfumer l'air ambiant il est nécessaire de placer, en outre, un bloc solide à base de naphtaline que l'on dispose environ à 1,50 m du sol par fixation par collage ou dans un récipient prévu à cet effet.

L'invention vise à pallier ces inconvénients. Un but de l'invention est de fournir un procédé de fabrication d'une composition parfumée, et plus particulièrement une solution aqueuse parfumée homogène et stable d'une essence de parfum, notamment de parfum naturel.

Un deuxième but de l'invention est de fournir un produit liquide pour chasse d'eau qui possède une rémanence du parfum dans l'enceinte des toilettes, à savoir que le parfum ou le mélange de parfums se diffuse au cours du temps dans l'atmosphère ambiante de l'enceinte des toilettes.

Un autre but de l'invention est de fournir un produit liquide pour chasse d'eau qui possède conjointement les propriétés suivantes :

- une homogénéité et une stabilité dans le temps
- un pouvoir désinfectant
- une solubilisation du parfum sans relargage dans le temps.

Or, de manière surprenante, on a constaté que lorsque l'on solubilise une essence de parfum avec un émulgateur, conjointement avec un alcool, on peut solubiliser l'essence de parfum naturel dans l'eau, la solution restant homogène. Enfin, on obtient une rémanence de la fragrance

L'invention concerne donc un procédé de fabrication d'une composition parfumée, caractérisé par le fait que l'on solubilise une essence de parfum dans un mélange d'alcool et d'un émulgateur puis que l'on ajoute de l'eau en vue de réaliser une solution aqueuse parfumée homogène et stable.

Par "essence de parfum naturel", on entend dans la présente description un extrait de plante pur, par exemple un extrait d'oeillet ou un extrait de plante et un fixateur ou un colorant permettant d'exalter le parfum. Le procédé selon l'invention permet d'obtenir une solution hydroalcoolique d'un parfum naturel, cette solution restant homogène. De préférence on utilise un émulgateur non ionique par exemple, un tensio-actif du type polymère à base d'oxyde d'éthylène sur noyau aromatique, plus préférentiellement un octylphénol à 10 moles d'oxyde d'éthylène.

L'alcool utilisé est plus préférentiellement de l'éthanol par exemple, de l'éthanol dénaturé.

On effectue la solubilisation du parfum en ajoutant à

l'essence de parfum naturel , au moins 10% en poids, d'alcool , à savoir au moins 10 % en poids d'éthanol dénaturé et au moins 30 % en poids, d'émulgateur.

Par exemple on ajoute à 40 % en poids d'essence de parfum naturel, 15 % en poids d'éthanol dénaturé et 35 % en poids d'émulgateur non ionique.

Selon l'invention, on utilise avec le colorant anionique acide, un désinfectant amphotère. De préférence, la composition selon l'invention contient au moins 5 % en poids de désinfectant amphotère. Le désinfectant amphotère utilisé est de préférence un dérivé amphotère d'ammonium quaternaire, par exemple un composé de formule (I) suivante :

$$(I) \qquad \left( R - \underset{\displaystyle CH_3}{\overset{\displaystyle CH_3}{\underset{|}{\overset{|}{N}}}} - CH_2 - COONa \right)^+ \qquad Cl^-$$

R étant un radical alkyle en $C_{13}$- $C_{15}$ ( de 13 à 15 atomes de carbone). Ce composé est un tensio-actif bactéricide algicide.

Le désinfectant dérivé amphotère est introduit dans la solution dans des proportions variant de 5 à 10 % en poids, et on introduit, en outre, de 0,5 à 2% d'un séquestrant qui est un sel d'éthylène diamine tétracétique.

Enfin, le colorant utilisé est un colorant acide, notamment n'ayant pas d'affinité avec l'émail. De préférence ce colorant est le bleu sulfacide 6J ayant au "color index" le N° 42045 Acide Blue I qui est un colorant acide du triphénylméthane.

Ainsi la composition selon l'invention comprend plus préférentiellement, en outre, au moins un colorant dans des proportions de 0 à 5 % et pour un fixateur, dans des proportions variant de 0 à 10 %.

Bien entendu, selon l'invention on peut utiliser dans les solutions aucun colorant ou tout colorant acide, sa couleur pouvant être bleue, rose, verte, etc ... selon les habitudes des utilisateurs. Par exemple, pour un produit utilisé en France, on préfère le bleu sulfacide 6J, tandis que pour un produit utilisé aux Etats-Unis d'Amérique, on pourra utiliser un colorant acide rose.

Aussi, la composition finalement obtenue comprend notamment de 2 à 5 % d'éthanol, de 5 à 6 % d'émulgateur, de 0,4 à 8 % d'une essence naturelle de parfum, de l'eau en quantité suffisante.

La description suivante, en regard de l'exemple annexé à titre non limitatif, permettra de comprendre comment l'invention peut être mise en pratique.

Exemple :

On prépare une solution alcoolique de parfum en utilisant, en poids, :

- 40 % de parfum à base d'essences naturelles
- 35 % d'un octyl phénol à 10 moles d'oxyde d'éthylène
- 25 % d'éthanol dénaturé.

Puis on prépare la composition pour chasse d'eau en effectuant le mélange de :

- 73,3 % d'eau
- 5,0 % d'un dérivé amphotère d'ammonium quaternaire de formule (I)
- 1,2 % de bleu sulfacide 6J
- 15 % de la solution alcoolique de parfum
- (soit 6 % de parfum pur à base d'essences naturelles
  5,25 % d'octyl phénol,
  3,25 % d'éthanol).

et,

0,5 % d'un séquestrant organique, de préférence un sel d'éthylène diamine tétracétique, les pourcentages étant exprimés en poids.

## REVENDICATIONS

1/ Procédé de fabrication d'une composition parfumée, caractérisé par le fait que l'on solubilise une essence de parfum dans un mélange d'alcool et d'un émulgateur, puis on ajoute de l'eau en vue de réaliser une solution aqueuse parfumée homogène et stable.

2/ Composition fabriquée selon le procédé de la revendication 1, caractérisée par le fait qu'elle comprend au moins une essence naturelle de parfum, solubilisée par un émulgateur et un alcool.

3/ Composition selon la revendication 2, caractérisée par le fait qu'elle comprend, en outre, au moins un colorant acide notamment n'ayant pas d'affinité pour l'émail.

4/ Composition selon l'une quelconque des revendications 2 et 3, caractérisée par le fait que l'alcool est l'éthanol.

5/ Composition selon l'une quelconque des revendications 1 à 4, caractérisée par le fait que l'essence de parfum est solubilisée par au moins 30% en poids d'émulgateur et au moins 10% en poids d'alcool.

6/ Composition selon l'une quelconque des revendications 2 à 5, caractérisée par le fait que l'émulgateur est un octylphénol à 10 moles d'oxyde d'éthylène

7/ Composition selon l'une quelconque des revendications 2 à 6, caractérisée par le fait qu'elle contient un désinfectant amphotère.

8/ Composition selon la revendication 7, caractérisée par le fait qu'elle contient au moins 5% en poids de désinfectant amphotère.

9/ Composition selon l'une quelconque des revendications 2 à 8, caractérisée par le fait que le désinfectant amphotère est un ammonium quaternaire de formule (I).

$$ \text{(I)} \quad (R - \overset{CH_3}{\underset{CH_3}{N}} - CH_2 - COON_a)^+ \qquad Cl^- $$

R étant un radical alkyle en $C_{13}$-$C_{15}$.

10/ Composition selon l'une quelconque des revendications 2 à 9 caractérisée par le fait qu'elle comprend :

. de 2 à 5 % d'éthanol,
. de 5 à 6 % d'émulgateur,
. de 0,4 à 8 % d'une essence naturelle de parfum,
. de l'eau en quantité suffisante.

11/ Composition selon l'une quelconque des revendications 2 à 10, caractérisée par le fait qu'elle comprend, en outre, au moins un colorant de 0 à 5 %, notamment de bleu sulfacide 6J et/ou un fixateur de 0 à 10 %.

12/ Composition selon l'une quelconque des revendications 2 à 11, caractérisée par le fait qu'elle comprend, en outre de 5 à 10 % d'un dérivé amphotère de formule (I) et de 0,5 à 2 % d'un sel d'éthylène diamine tétracétique.